(19) Europäisches Patentamt
European Patent Office
Office européen des brevets

(11) EP 3 782 660 A1

(12) # EUROPEAN PATENT APPLICATION

(43) Date of publication:
**24.02.2021 Bulletin 2021/08**

(21) Application number: **19193369.6**

(22) Date of filing: **23.08.2019**

(51) Int Cl.:
***A61L 31/06*** *(2006.01)* ***C08G 64/12*** *(2006.01)*
***C08L 69/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **SABIC Global Technologies B.V.**
**4612 PX Bergen op Zoom (NL)**

(72) Inventors:
- **DE BROUWER, Johannes**
**4612 PX Bergen op Zoom (NL)**
- **VAN DE GRAMPEL, Robert Dirk**
**4612 PX Bergen op Zoom (NL)**
- **VAN DER MEE, Mark Adrianus Johannes**
**4612 PX Bergen op Zoom (NL)**
- **DIMATTIA, Peter**
**4612 PX Bergen op Zoom (NL)**

(74) Representative: **Elzaburu S.L.P.**
**Miguel Angel 21, 2nd floor**
**28010 Madrid (ES)**

(54) **POLYCARBONATE COMPOSITIONS FOR MEDICAL DEVICES**

(57) A thermoplastic composition including a copolycarbonate comprising bisphenol A carbonate units and second carbonate units of the formula

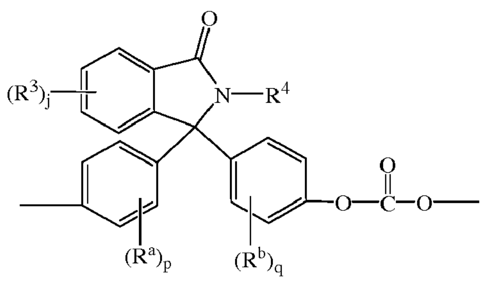

wherein $R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-12}$ alkyl, $R^4$ is hydrogen, $C_{2-6}$ alkyl or phenyl optionally substituted with 1 to 5 $C_{1-6}$ alkyl groups, p, q, and j are each independently 0 to 4, optionally a bisphenol A homopolycarbonate; and an acid stabilizer comprising a sulfonic acid ester; wherein the composition has an improved volatile or non-volatile organic compound content and yellowness index value when compared to a reference sample of an otherwise identical composition except for not containing the stabilizer.

EP 3 782 660 A1

## Description

### BACKGROUND

Extractables and leachables are components that are present in a material which may migrate from the material (typically a form of packaging) into a contained product (typically food, medicine, pharmaceuticals). It is important to minimize the levels of such components in order not to degrade the quality of the packaged product, and meet industry guidelines. Characterization efforts of extractables and leachables can be time consuming, costly and may result in disqualification of a material, e.g. a plastic material, for its intended use. Hence, an industry need are improved materials with no, or a very low levels of extractables and leachables, especially in the case of medical end-use applications or equipment that interact with the humans and animals, such as drug delivery systems. In addition, materials that have good heat stability and low levels of extractables and leachables would be beneficial for medical end-uses/equipment that are subject to sterilization and/or heat exposure and/or frictional force.

Some known "high heat" copolycarbonates can have high glass transition temperatures of 150 °C or higher. But such polycarbonates are typically more yellow after processing and have lower transmission values. Recently, International Application Publication WO 2017/203496 presented the copolycarbonate based thermoplastic compositions exhibiting desirable high heat properties. There still however remains a need for polycarbonate compositions having improved balance of high heat performance and optical properties with limited extractables and leachables making them suitable for packaging applications.

### SUMMARY

The present disclosure provides for a drug delivery system containing a housing with a high heat plastic material. As an embodiment of this disclosure, the present disclosure provides for a drug delivery system comprising a housing, wherein the housing contains a thermoplastic composition comprising: a copolycarbonate comprising bisphenol A carbonate units and second carbonate units of the formula

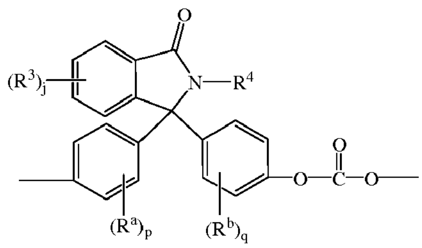

wherein $R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4; and optionally, a bisphenol A homopolycarbonate; wherein one or more of the following conditions apply: the bisphenol A carbonate units in the copolycarbonate are derived from a bisphenol A monomer having a purity of equal to or greater than 99.70%; or the bisphenol A homopolycarbonate is present in an amount of greater than zero percent and is derived from a bisphenol A monomer having a purity of equal to or greater than 99.70%; or the bisphenol A purity of the thermoplastic composition is equal to or greater than 99.60%; and wherein the composition has: a volatile or non-volatile organic compound content that is at least 30% lower than a reference sample of an otherwise identical composition in which none of conditions (i)-(iii) applies, a glass transition temperature of 155 °C or higher, preferably 155 °C to 280 °C, and a molded part of the composition with a thickness of 2.5 millimeter (mm) has a yellowness index (YI) determined according to ASTM D1925 at least 30% lower as compared to a reference sample of an otherwise identical composition except none of conditions (i)-(iii) applies, when both the sample and the reference sample are molded at a temperature of equal to or greater than 330 °C.

The present disclosure further relates to a drug delivery system comprising a housing, wherein the housing contains a thermoplastic composition comprising: bisphenol-A polycarbonate, wherein a molded article of the composition has transmission level greater than or equal to 90.0% at 2.5 mm thickness as measured by ASTM D1003-00 and a yellow index (YI) less than or equal to 1.5 as measured by ASTM D1925; and optionally a second polycarbonate derived from bisphenol-A, wherein the second polycarbonate is different than the bisphenol-A polycarbonate.

In an embodiment, a thermoplastic composition comprises: a copolycarbonate comprising bisphenol A carbonate units and second carbonate units of the formula

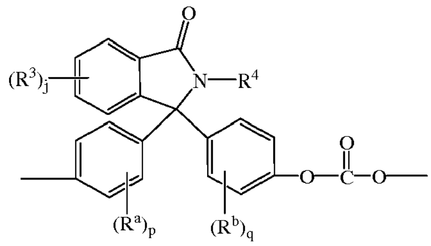

wherein $R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4, optionally a bisphenol A homopolycarbonate; and an acid stabilizer comprising a sulfonic acid ester; wherein the composition has: a volatile or non-volatile organic compound content that is at least 30% lower than a reference sample of an otherwise identical composition in which none of conditions (i)-(iii) applies, a glass transition temperature of 155 °C or higher; a molded part of the composition with a thickness of 2.5 mm has a yellowness index determined according to ASTM D1925 at least 30% lower as compared to a reference sample of an otherwise identical composition except for not containing the stabilizer, when both the sample and the reference sample are molded at a temperature of equal to or greater than 330 °C.

In another aspect, an article comprises the above-described thermoplastic composition.

In still another aspect, a method of manufacture of an article comprises molding, extruding, or shaping the above-described thermoplastic composition into an article.

The above described and other features are exemplified by the following drawings, detailed description, examples, and claims.

DETAILED DESCRIPTION

Conventional plastics used in packaging may include certain materials that can migrate from the plastic material into the item contained therein. It is desirable that these materials, known as extractables and leachables, are minimized so that the quality of the packaged product is not degraded. Compositions of the present disclosure provide polycarbonate materials for containers as well as components of drug delivery systems. It has been found that a high optical quality polycarbonate may be useful as for molding plastic objects comprising fewer and/or lower levels of extractables and leachables. The disclosed composition may comprise a copolycarbonate comprising bisphenol A carbonate units and a second carbonate, and optionally a BPA homopolycarbonate. The composition may exhibit a glass transition temperature of 155 °C or higher, preferably 155 °C to 280 °C, and a molded part formed from the composition with a thickness of 2.5 mm has a yellowness index (YI) determined according to ASTM D1925 at least 30% lower as compared to a reference sample of an otherwise identical composition except none of conditions (i)-(iii) applies, when both the sample and the reference sample are molded at a temperature of equal to or greater than 330 °C.

Specifically, the composition may comprise a copolycarbonate comprising bisphenol A carbonate units and second carbonate units of the formula

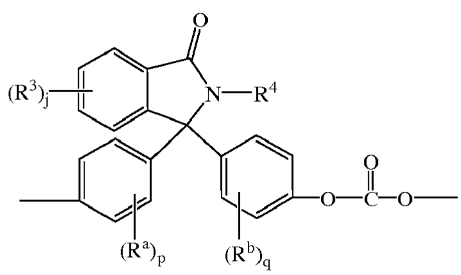

wherein

- $R^a$ and $R^b$ are each independently a $C_{1-12}$ alkyl, $C_{1-12}$ alkenyl, $C_{3-8}$ cycloalkyl, or $C_{1-12}$ alkoxy,
- each $R^3$ is independently a $C_{1-6}$ alkyl,
- $R^4$ is hydrogen, $C_{2-6}$ alkyl or phenyl optionally substituted with 1 to 5 $C_{1-6}$ alkyl groups,
- p, q, and j are each independently 0 to 4; and

optionally, a bisphenol A homopolycarbonate;

wherein one or more of the following conditions apply: the bisphenol A carbonate units in the copolycarbonate are derived from a bisphenol A monomer having a purity of equal to or greater than 99.70%; or the bisphenol A homopolycarbonate is present in an amount of greater than zero percent and is derived from a bisphenol A monomer having a purity of equal to or greater than 99.70%; or the bisphenol A purity of the thermoplastic composition is equal to or greater than 99.60%.

As an example, the composition may comprise a phthalimidine copolycarbonate. As used herein, phthalimidine copolycarbonates are high heat copolycarbonates having a glass transition temperature of 155°C or higher, and comprising bisphenol A carbonate units and second carbonate units of formula (1)

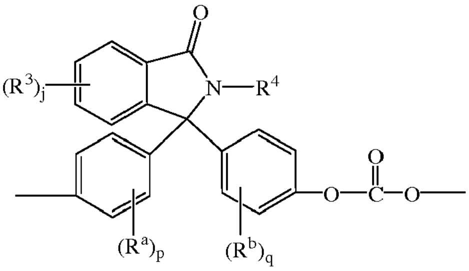

(1)

wherein $R^a$ and $R^b$ are each independently a $C_{1-12}$ alkyl, $C_{1-12}$ alkenyl, $C_{3-8}$ cycloalkyl, or $C_{1-12}$ alkoxy, preferably a $C_{1-3}$ alkyl, each $R^3$ is independently a $C_{1-6}$ alkyl, $R^4$ is hydrogen, $C_{1-6}$ or $C_{2-6}$ alkyl or phenyl optionally substituted with 1 to 5 $C_{1-6}$ alkyl groups, and p and q are each independently 0 to 4, preferably 0 to 1. For example, the second carbonate units can be of formula (1a)

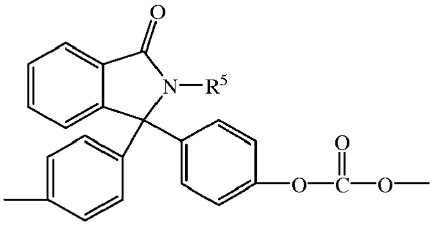

(1a)

wherein $R^5$ is hydrogen, phenyl optionally substituted with up to five $C_{1-6}$ alkyl groups, or $C_{1-4}$ alkyl, such as methyl or $C_{2-4}$ alkyl. In an aspect, $R^5$ is hydrogen or phenyl, preferably phenyl. Carbonate units (1a) wherein $R^5$ is phenyl can be derived from 2-phenyl-3,3'-bis(4-hydroxy phenyl)phthalimidine (also known as 3,3-bis(4-hydroxyphenyl)-2-phenylisoindolin-1-one or N-phenyl phenolphthalein or "PPPBP").

Bisphenol A carbonate units have the formula (2).

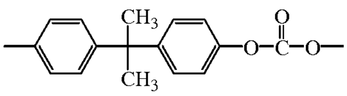

(2)

In some aspects, the high heat copolycarbonates further include third carbonate units different from bisphenol A carbonate units and second carbonate units. The third carbonate units can have the formula (3), (4), or (5),

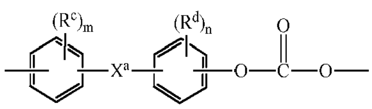

(3),

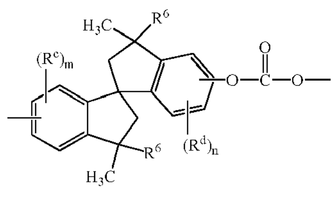

(4), or

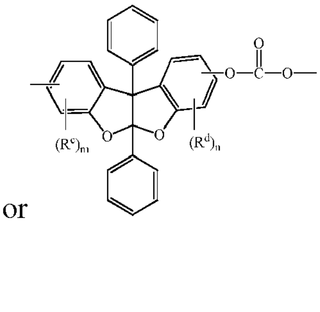

(5),

wherein $R^c$ and $R^d$ are each independently a $C_{1-12}$ alkyl, $C_{1-12}$ alkenyl, $C_{3-8}$ cycloalkyl, or $C_{1-12}$ alkoxy, each $R^6$ is independently $C_{1-3}$ alkyl or phenyl, preferably methyl, $X^a$ is a $C_{6-12}$ polycyclic aryl, $C_{3-18}$ mono- or polycycloalkylene, $C_{3-18}$ mono- or polycycloalkylidene, $-(Q^1)_x-G-(Q^2)_y-$ group wherein $Q^1$ and $Q^2$ are each independently a $C_{1-3}$ alkylene, G is a $C_{3-10}$ cycloalkylene, x is 0 or 1, and y is 1, or $-C(P^1)(P^2)-$ wherein $P^1$ is $C_{1-12}$ alkyl and $P^2$ is $C_{6-12}$ aryl; and m and n are each independently 0 to 4.

Exemplary third carbonate units include the following:

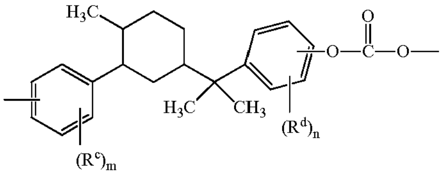 (3a), 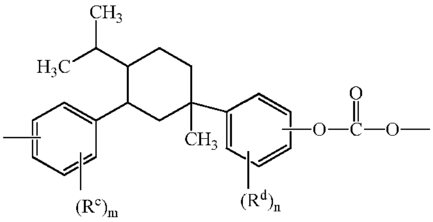 (3b),

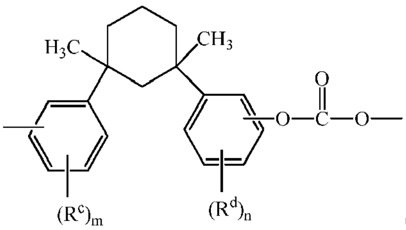 (3c), 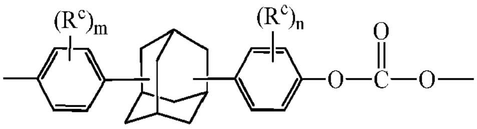 (3d),

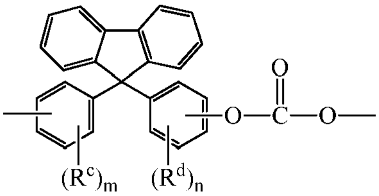 (3i), 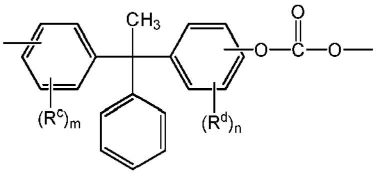 (3j),

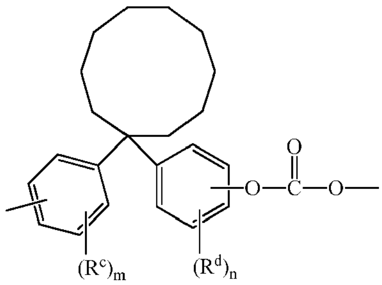 (3e), 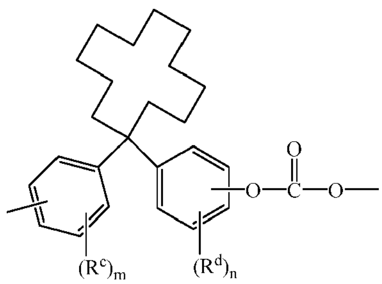 (3f)

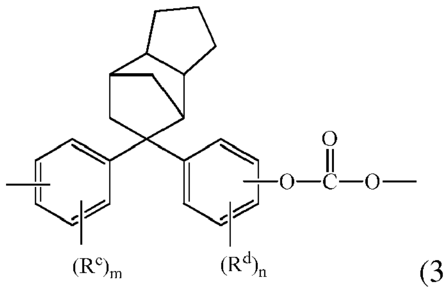 (3g), or 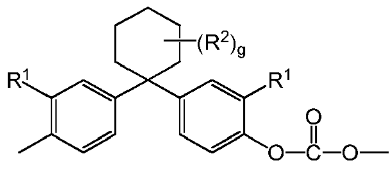 (3h),

wherein $R^c$ and $R^d$ are the same as defined herein for formulas (3)-(5), each $R^1$ is independently hydrogen or $C_{1-4}$ alkyl, each $R^2$ is independently $C_{1-4}$ alkyl, and g is 0 to 10. In a specific aspect the third carbonate units are 1,1-bis(4-hydroxyphenyl)-3,3,5-trimethylcyclohexane carbonate units, 1,1-bis(4-hydroxy-3-methylphenyl)cyclohexane, or a combination thereof. Preferably, the third carbonate units are 1,1-bis(4-hydroxyphenyl)-3,3,5-trimethyl-cyclohexane (BPA TMC) carbonate units.

In various aspects, the disclosed composition may comprise a copolycarbonate comprising from 15 mole percent (mol%) to 90 mole % of the bisphenol A carbonate units and 10 mol% to 85 mol% of the second carbonate units, each based on the total number of carbonate units in the copolycarbonate.

When the third carbonate units are present, the copolycarbonates can comprise 10 mol% to 70 mol% of the bisphenol A carbonate units, 5 mol% to 50 mol% of the second carbonate units, and 5 mol% to 50 mol% of the third carbonate units, each based on the sum of moles of the bisphenol A carbonate units, second carbonate units, and third carbonate units. Preferably, the copolycarbonates comprise 30 mol% to 60 mol% of the bisphenol A carbonate units, 5 mol% to 35 mol% of the second carbonate units, 5 mol% to 35 mol% of the third carbonate units, each based on the sum of the moles of the bisphenol A carbonate units, second carbonate units, and third carbonate units.

The disclosed copolycarbonates may be highly random copolymers, which have less than 15 mol% or less than 10 mol% of the second carbonate units directly coupled to another second carbonate unit based on the total number of carbonate units in the copolycarbonates. The molar percent can be determined by nuclear magnetic resonance spectroscopy (NMR). Without wishing to be bound by theory, it is believed that by keeping the randomness of the high heat polymer, the properties of the high heat polymer remain consistent from batch to batch.

The high heat copolycarbonates described herein have a weight average molecular weight of 10,000 to 50,000 Daltons, preferably 16,000 to 30,000 Daltons, as measured by gel permeation chromatography (GPC), using a crosslinked styrenedivinylbenzene column and calibrated to bisphenol A homopolycarbonate references. GPC samples are prepared at a concentration of 1 mg per ml, and are eluted at a flow rate of 1.5 milliliter (ml) per minute.

The composition may have a high glass transition temperature ($T_g$). The $T_g$ of the high heat copolycarbonates is 155 to 280 °C, or from about 165 to 260 °C, or from about 185 to 230 °C, determined by differential scanning calorimetry (DSC) as per ASTM D3418 with a 20 °C/min heating rate.

The high heat copolycarbonates can have high heat resistance. The heat deflection temperature (HDT) of the high heat copolycarbonates is 145 to 270 °C, more preferably 155 to 260°C, even more preferably 175 to 220 °C, measured flat on a 80 x 10 x 4 millimeter (mm) bar with a 64 mm span at 0.45 MPa according to ISO 75/Bf.

The high heat copolycarbonates can have a high Vicat softening temperature. In an aspect, the high heat copolycarbonates have a Vicat B120 of 150 to 275 °C, preferably 160 to 255 °C, even more preferably 180 to 225 °C, measured according to ISO 306.

The high heat copolycarbonates can be present in an amount of 10 wt.% to 99 wt.%, of 20 wt.% to 80 wt.%, of 40 wt.% to 70 wt.%, or of 50 wt.% to 70 wt.% based on the total weight of the compositions.

The high heat polycarbonates are essentially free of certain metal ions, anions, and low molecular weight molecules (fewer than 150 grams per mol, g/mol). Such levels of these metal ions may further enhance the optical properties of the thermoplastic compositions, In an aspect, the copolycarbonates comprise less than 2 ppm of each of triethyl amine, calcium ions, magnesium ions, potassium ions, iron ions, and chloride ions.

Optionally, the compositions include a bisphenol A homopolycarbonate. The composition may comprise from about 10 to 90 wt.% of the bisphenol A homopolycarbonate based on the total weight of the composition. In certain aspects, the composition may comprise a bisphenol A homopolycarbonate having specific additional properties. The bisphenol A homopolymer carbonate can be derived from a bisphenol A monomer having a purity equal to or greater than 99.6% determined by HPLC. In an aspect, the bisphenol A homopolycarbonate is manufactured via an interfacial process using a BPA monomer having both a high level of organic purity. For example, the BPA homopolycarbonate may be derived from a BPA monomer having a purity of equal to or greater than 99.7% (measured by HPLC).

The BPA polycarbonate may have a sulfur level of less than or equal to 2 parts per million (ppm) as measured by a commercially available Total Sulfur Analysis based on combustion and coulometric detection. The organic purity can be defined as 100 wt.% minus the sum of known and unknown impurities detected using ultraviolet (UV) (see HPLC method in Nowakowska et al., Polish J. Appl. Chem., XI(3), 247-254 (1996)). In addition, an end-capping agent is present during manufacture of the bisphenol A homopolycarbonate such that bisphenol A homopolycarbonate comprises a free hydroxyl level less than or equal to 150 ppm. Bisphenol A homopolycarbonates of high purity, suitable for use in the present compositions, can also be manufactured via the melt process.

In an aspect, the bisphenol A polycarbonate homopolymer is a linear bisphenol A polycarbonate homopolymer having a weight average molecular weight of 10,000 to 100,000 Daltons, specifically 15,000 to 50,000 Daltons, more specifically 17,000 to 35,000 Daltons, as measured by gel permeation chromatography (GPC), using a crosslinked styrenedivinylbenzene column and calibrated to polycarbonate references. GPC samples are prepared at a concentration of 1 mg per ml, and are eluted at a flow rate of 1.5 ml per minute.

More than one bisphenol A polycarbonate homopolymer can be present. For example, the polycarbonate compositions can comprise a first bisphenol A polycarbonate homopolymer having a weight average molecular weight of 20,000 Daltons to 25,000 Daltons as measured by GPC using BPA polycarbonate standards and a second bisphenol

A polycarbonate homopolymer having a weight average molecular weight of 28,000 to 32,000 Daltons, or a second bisphenol A polycarbonate homopolymer having a weight average molecular weight of 16,000 Daltons to 20,000 Daltons, each measured by GPC using BPA polycarbonate standards. The weight ratio of the first bisphenol A polycarbonate homopolymer relative to the second bisphenol A polycarbonate homopolymer is 10:1 to 1:10, specifically 5:1 to 1: 5, more specifically 3:1 to 1:3 or 2:1 to 1:2.

The polycarbonate homopolymer can be present in an amount of 10 wt.% to 90 wt.%, preferably 20 wt.% to 80 wt.% or 30 wt.% to 60 wt.% based on the total weight of the thermoplastic composition.

In an aspect, the bisphenol A purity of the thermoplastic composition is equal to or greater than 99.60% measured using HPLC. As used herein, the bisphenol A purity of the thermoplastic composition refers to the overall purity of the bisphenol A monomer used to prepare the high heat copolycarbonate and the bisphenol A homopolymer, if present. The bisphenol A purity of a thermoplastic composition can be determined by a mild depolymerization followed by a HPLC analysis. For example, about 200 milligrams (mg) of the thermoplastic composition is dissolved in 5 ml of tetrahydrofuran (THF) and 2 ml of a 10% solution of potassium hydroxide diluted in methanol. The depolymerization of polycarbonate is carried out with the use of these solvents. The solution is shaken for 2 hours. Then, 2 milliliters (ml) of acetic acid are added to protonate the BPA carbonate salts and decrease the pH. The solution is shaken again for half an hour for homogenization and dissolution of all precipitates. The sample is analyzed by HPLC. The wt% of BPA impurities in the thermoplastic composition can be calculated by:

$$\text{wt\% of impurities in BPA} = \frac{\text{wt\% of impurities} * 254}{228} \quad \text{(equation 1)}.$$

In equation 1, wt% of impurities refers to the impurities percent measured by HPLC after depolymerization. Because the BPA molar mass is different from the carbonated BPA, the wt% of impurities is multiplied by 254 grams per mole (g/mol) and divided by 228 g/mol. 254 g/mol corresponds to the BPA carbonate molar mass and the BPA molar mass is equal to 228 g/mol.

The polycarbonates described herein can be manufactured by processes such as interfacial polymerization and melt polymerization, which are known, and are described, for example, in WO 2013/175448 A1 and WO 2014/072923 A1. An end-capping agent (also referred to as a chain stopper agent or chain terminating agent) can be included during polymerization to provide end groups, for example monocyclic phenols such as phenol, p-cyanophenol, and $C_1$-$C_{22}$ alkyl-substituted phenols such as p-cumyl-phenol, resorcinol monobenzoate, and p-and tertiary-butyl phenol, monoethers of diphenols, such as p-methoxyphenol, monoesters of diphenols such as resorcinol monobenzoate, functionalized chlorides of aliphatic monocarboxylic acids such as acryloyl chloride and methacryoyl chloride, and mono-chloroformates such as phenyl chloroformate, alkyl-substituted phenyl chloroformates, p-cumyl phenyl chloroformate, and toluene chloroformate. Combinations of different end groups can be used. Branched polycarbonate blocks can be prepared by adding a branching agent during polymerization, for example trimellitic acid, trimellitic anhydride, trimellitic trichloride, tris-p-hydroxyphenylethane, isatin-bis-phenol, tris-phenol TC (1,3,5-tris((p-hydroxyphenyl)isopropyl)benzene), tris-phenol PA (4(4(1,1-bis(p-hydroxyphenyl)-ethyl) alpha, alpha-dimethyl benzyl)phenol), 4-chloroformyl phthalic anhydride, trimesic acid, and benzophenone tetracarboxylic acid. The branching agents can be added at a level of 0.05 to 2.0 wt. %. Combinations comprising linear polycarbonates and branched polycarbonates can be used.

One or more acid additives may be included in the disclosed composition. The acid additive may act as a stabilizer and may improve certain properties of the composition including optical and aesthetic properties. Surprisingly it has been found that including a sulfonic acid ester in a thermoplastic composition containing the high heat copolycarbonate and the optional bisphenol A homopolycarbonate simultaneously improves the initial color of the thermoplastic composition and the color stability of the composition after the composition is molded under aggressive conditions and/or after the composition is aged at an elevated temperature for a prolonged period of time. For example, a molded part of the composition with a thickness of 2.5 mm has a yellowness index (YI) determined according to ASTM D1925 at least 30% lower as compared to a reference sample of an otherwise identical composition except for not containing the stabilizer, when both the sample and the reference sample are molded at a temperature of equal to or greater than 330 °C. In another aspect, a molded article of the composition, when tested at thickness of 2.5 mm determined according to ASTM D1925, has a change in yellowness index of less than 20, preferably less than 10, more preferably less than 5, following molding under aggressive conditions as compared to a reference article of an identical composition molded under standard process conditions. As used herein, aggressive molding conditions include a molding temperature of equal to or greater than 330 °C, and standard molding conditions include a molding temperature of less than 330 °C.

Moreover, the improvement on the color and color stability provided by the inclusion of the sulfonic acid ester is more significant than the improvement provided by other acid additives including acid stabilizers such as phosphorous acid $H_3PO_3$.

Acid stabilizers can include an ester of a $C_{6-30}$ aryl, $C_{7-30}$ aralkyl or $C_{1-30}$ alkyl sulfonic acid, or a combination

thereof. The acid stabilizers can be represented by Formula (6)

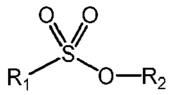 (6)

wherein $R_1$ is a $C_{6-30}$ aryl, $C_{7-30}$ arylalkylene or $C_{1-30}$ alkyl, and $R_2$ is $C_{1-30}$ alkyl. Preferably, $R_1$ is a $C_{6-12}$ aryl and $R_2$ is $C_{1-10}$ alkyl. In an aspect, the stabilizer is an ester of a $C_{6-12}$ aryl sulfonic acid, preferably the stabilizer is an ester of p-toluenesulfonic acid, and more preferably the stabilizer is butyl tosylate.

The acid stabilizer can be used in an amount of up to 40 ppm, preferably 1 to 40 ppm, more preferably 2 to 20 ppm, and still more specifically 4 to 15 ppm, by weight based on the total weight of the thermoplastic composition. Use of greater amounts of acid stabilizer does not improve molding or heat aging stability, but can significantly adversely affect hydro aging or autoclaving stability of the thermoplastic composition.

The thermoplastic composition can also contain an epoxy additive. The inclusion of an epoxy compound can further improve color stability, or molecular weight stability of the thermoplastic composition after hydro aging or autoclaving. Epoxy compounds useful as additives include epoxy modified acrylic oligomers or polymers (such as a styrene-acrylate-epoxy polymer, prepared from for example a combination of: a substituted or unsubstituted styrene such as styrene or 4-methylstyrene; an acrylate or methacrylate ester of a $C_{1-22}$ alkyl alcohol such as methyl acrylate, methyl methacrylate, ethyl acrylate, butyl acrylate, or the like; and an epoxy-functionalized acrylate such as glycidyl acrylate, glycidyl methacrylate, 2-(3,4-epoxycyclohexyl)ethyl acrylate, 2-(3,4-epoxycyclohexyl)ethyl methacrylate, or the like), or an epoxy carboxylate oligomer based on cycloaliphatic epoxides (such as, for example, 3,4-epoxycyclohexylmethyl-3,4-epoxycyclohexylcarboxylate, or the like). Specific commercially available exemplary epoxy functionalized stabilizers include Cycloaliphatic Epoxide Resin ERL-4221 supplied by Union Carbide Corporation (a subsidiary of Dow Chemical), Danbury, CT; and epoxy modified acrylates such as JONCRYL™ ADR-4300 and JONCRYL™ ADR-4368, available from Johnson Polymer Inc., Sturtevant, WI. Epoxy additives can be used in amounts of up to 1 wt%, specifically 0.001 to 1 wt%, more specifically 0.001 to 0.5 wt%, based on the total weight of the thermoplastic composition. In an aspect, the epoxy additive can be included in an amount of 0.001 to 0.3 wt%, specifically 0.01 to 0.3 wt%, and more specifically 0.1 to 0.3 wt%, based on the total weight of the thermoplastic composition. Use of greater amounts of epoxy compound may cause more splay, i.e., mold lines which fan outward from the point of injection into the mold, and observable to the unaided eye in molded articles comprising the thermoplastic composition.

The thermoplastic compositions can include various additives ordinarily incorporated into polymer compositions of this type, with the proviso that the additive(s) are selected so as to not significantly adversely affect the desired properties of the thermoplastic composition, in particular melt flow, thermal, transparency, and surface properties. Such additives can be mixed at a suitable time during the mixing of the components for forming the composition. Additives include fillers, reinforcing agents, antioxidants, heat stabilizers, light stabilizers, ultraviolet (UV) light stabilizers, plasticizers, lubricants, mold release agents, antistatic agents, colorants such as such as titanium dioxide, carbon black, and organic dyes, surface effect additives, radiation stabilizers, flame retardants, anti-drip agents, and impact modifiers. In an aspect, the thermoplastic composition further comprises a processing aid, a heat stabilizer, an ultraviolet light absorber, a colorant, a flame retardant, an impact modifier, or a combination thereof. A combination of additives can be used, for example a combination of a heat stabilizer, mold release agent, and ultraviolet light stabilizer. In general, the additives are used in the amounts generally known to be effective. For example, the total amount of the additives (other than any impact modifier, filler, or reinforcing agents) can be 0 to 5 wt. % or 0.01 to 5 wt. %, based on the total weight of the thermoplastic composition.

Antioxidant additives include organophosphites such as tris(nonyl phenyl)phosphite, tris(2,4-di-t-butylphenyl)phosphite, bis(2,4-di-t-butylphenyl)pentaerythritol diphosphite, distearyl pentaerythritol diphosphite; alkylated monophenols or polyphenols; alkylated reaction products of polyphenols with dienes, such as tetrakis[methylene(3,5-di-tert-butyl-4-hydroxyhydrocinnamate)] methane; butylated reaction products of para-cresol or dicyclopentadiene; alkylated hydroquinones; hydroxylated thiodiphenyl ethers; alkylidenebisphenols; benzyl compounds; esters of beta-(3,5-di-tert-butyl-4-hydroxyphenyl)-propionic acid with monohydric or polyhydric alcohols; esters of beta-(5-tert-butyl-4-hydroxy-3-methylphenyl)-propionic acid with monohydric or polyhydric alcohols; esters of thioalkyl or thioaryl compounds such as distearylthiopropionate, dilaurylthiopropionate, ditridecylthiodipropionate, octadecyl-3-(3,5-di-tert-butyl-4-hydroxyphenyl)propionate, pentaerythrityl-tetrakis[3-(3,5-di-tert-butyl-4-hydroxyphenyl)propionate; amides of beta-(3,5-di-tert-butyl-4-hydroxyphenyl)-propionic acid, or combinations comprising at least one of the foregoing antioxidants. Antioxidants are used in amounts of 0.01 to 0.1 parts by weight, based on 100 parts by weight of the total composition, excluding any filler.

Heat stabilizer additives include organophosphites such as triphenyl phosphite, tris-(2,6-dimethylphenyl)phosphite, tris-(mixed mono-and di-nonylphenyl)phosphite; phosphonates such as dimethylbenzene phosphonate, phosphates such as trimethyl phosphate, or combinations comprising at least one of the foregoing heat stabilizers. Heat stabilizers are used in amounts of 0.01 to 0.1 parts by weight, based on 100 parts by weight of the total composition, excluding any filler.

Light stabilizers, including ultraviolet light (UV) absorbers, can also be used. Light stabilizers include benzotriazoles such as 2-(2-hydroxy-5-methylphenyl)benzotriazole and 2-(2-hydroxy-5-tert-octylphenyl)-benzotriazole, 2-hydroxy-4-n-octoxy benzophenone, or combinations comprising at least one of the foregoing light stabilizers. Light stabilizers are used in amounts of 0.01 to 5 parts by weight, based on 100 parts by weight of the thermoplastic composition, excluding any filler.

Flame retardants can also be used. Flame retardants include flame retardant salts such as alkali metal salts of perfluorinated $C_{1-16}$ alkyl sulfonates such as potassium perfluorobutane sulfonate (Rimar salt), potassium perfluoroctane sulfonate, tetraethylammonium perfluorohexane sulfonate, potassium diphenylsulfone sulfonate (KSS), and the like, sodium benzene sulfonate, sodium toluene sulfonate (NATS) and the like; and salts formed by reacting for example an alkali metal or alkaline earth metal (for example lithium, sodium, potassium, magnesium, calcium and barium salts) and an inorganic acid complex salt, for example, an oxo-anion, such as alkali metal and alkaline-earth metal salts of carbonic acid, such as $Na_2CO_3$, $K_2CO_3$, $MgCO_3$, $CaCO_3$, and $BaCO_3$ or fluoro-anion complex such as $Li_3AlF_6$, $BaSiF_6$, $KBF_4$, $K_3AlF_6$, $KAlF_4$, $K_2SiF_6$, and/or $Na_3AlF_6$ or the like. Rimar salt and KSS and NATS, alone or in combination with other flame retardants, are particularly useful in the compositions disclosed herein. Flame retardant salts are typically used in amounts of 0.01 to 1.0 parts by weight, based on 100 parts by weight of the thermoplastic composition.

Organophosphorus flame retardants can be used. Organophosphorus compounds include aromatic organophosphorus compounds having at least one organic aromatic group and at least one phosphorus-containing group, as well as organic compounds having at least one phosphorus-nitrogen bond.

In the aromatic organophosphorus compounds that have at least one organic aromatic group, the aromatic group can be a substituted or unsubstituted $C_{3-30}$ group containing one or more of a monocyclic or polycyclic aromatic moiety (which can optionally contain with up to three heteroatoms (N, O, P, S, or Si)) and optionally further containing one or more nonaromatic moieties, for example alkyl, alkenyl, alkynyl, or cycloalkyl. The aromatic moiety of the aromatic group can be directly bonded to the phosphorus-containing group, or bonded via another moiety, for example an alkylene group. The aromatic moiety of the aromatic group can be directly bonded to the phosphorus-containing group, or bonded via another moiety, for example an alkylene group. In an aspect the aromatic group is the same as an aromatic group of the polycarbonate backbone, such as a bisphenol group (e.g., bisphenol A), a monoarylene group (e.g., a 1,3-phenylene or a 1,4-phenylene), or a combination comprising at least one of the foregoing.

The phosphorus-containing group can be a phosphate ($P(=O)(OR)_3$), phosphite ($P(OR)_3$), phosphonate ($RP(=O)(OR)_2$), phosphinate ($R_2P(=O)(OR)$), phosphine oxide ($R_3P(=O)$), or phosphine ($R_3P$), wherein each R in the foregoing phosphorus-containing groups can be the same or different, provided that at least one R is an aromatic group. A combination of different phosphorus-containing groups can be used. The aromatic group can be directly or indirectly bonded to the phosphorus, or to an oxygen of the phosphorus-containing group (i.e., an ester).

In an aspect, the aromatic organophosphorus compound is a monomeric phosphate. Representative monomeric aromatic phosphates are of the formula $(GO)_3P=O$, wherein each G is independently an alkyl, cycloalkyl, aryl, alkylarylene, or arylalkylene group having up to 30 carbon atoms, provided that at least one G is an aromatic group. Two of the G groups can be joined together to provide a cyclic group. In some aspects G corresponds to a monomer used to form the polycarbonate, e.g., resorcinol. Exemplary phosphates include phenyl bis(dodecyl) phosphate, phenyl bis(neopentyl) phosphate, phenyl bis(3,5,5'-trimethylhexyl) phosphate, ethyl diphenyl phosphate, 2-ethylhexyl di(p-tolyl) phosphate, bis(2-ethylhexyl) p-tolyl phosphate, tritolyl phosphate, bis(2-ethylhexyl) phenyl phosphate, tri(nonylphenyl) phosphate, bis(dodecyl) p-tolyl phosphate, dibutyl phenyl phosphate, 2-chloroethyl diphenyl phosphate, p-tolyl bis(2,5,5'-trimethylhexyl) phosphate, 2-ethylhexyl diphenyl phosphate, and the like. A specific aromatic phosphate is one in which each G is aromatic, for example, triphenyl phosphate, tricresyl phosphate, isopropylated triphenyl phosphate, and the like.

Di- or polyfunctional aromatic phosphorus-containing compounds are also useful, for example, compounds of the formulas below

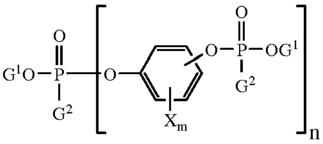 , 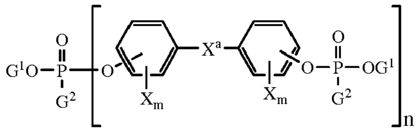 , and 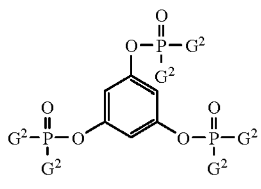

wherein each $G^1$ is independently a $C_{1-30}$ hydrocarbyl; each $G^2$ is independently a $C_{1-30}$ hydrocarbyl or hydrocarbyloxy; each X is independently a bromine or chlorine; m is 0 to 4, and n is 1 to 30. Di- or polyfunctional aromatic phosphorus-containing compounds of this type include resorcinol tetraphenyl diphosphate (RDP), the bis(diphenyl) phosphate of

hydroquinone and the bis(diphenyl) phosphate of bisphenol A, respectively, their oligomeric and polymeric counterparts, and the like.

Specific aromatic organophosphorus compounds have two or more phosphorus-containing groups, and are inclusive of acid esters of formula (8)

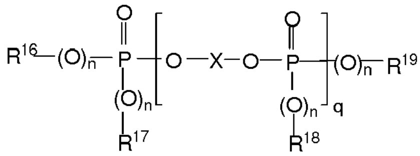

(8)

wherein $R^{16}$, $R^{17}$, $R^{18}$, and $R^{19}$ are each independently $C_{1-8}$ alkyl, $C_{5-6}$ cycloalkyl, $C_{6-20}$ aryl, or $C_{7-12}$ arylalkylene, each optionally substituted by $C_{1-12}$ alkyl, specifically by $C_{1-4}$ alkyl and X is a mono- or poly-nuclear aromatic $C_{6-30}$ moiety or a linear or branched $C_{2-30}$ aliphatic radical, which can be OH-substituted and can contain up to 8 ether bonds, provided that at least one of $R^{16}$, $R^{17}$, $R^{18}$, $R^{19}$, and X is an aromatic group. In some aspects $R^{16}$, $R^{17}$, $R^{18}$, and $R^{19}$ are each independently $C_{1-4}$ alkyl, naphthyl, phenyl($C_{1-4}$)alkylene, or aryl groups optionally substituted by $C_{1-4}$ alkyl. Specific aryl moieties are cresyl, phenyl, xylenyl, propylphenyl, or butylphenyl. In some aspects X in formula (8) is a mono- or poly-nuclear aromatic $C_{6-30}$ moiety derived from a diphenol. Further in formula (8), n is each independently 0 or 1; in some aspects n is equal to 1. Also in formula (8), q is from 0.5 to 30, from 0.8 to 15, from 1 to 5, or from 1 to 2. Specifically, X can be represented by the following divalent groups (9), or a combination comprising one or more of these divalent groups.

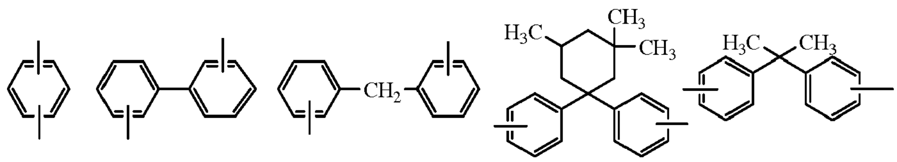

(9)

In these aspects, each of $R^{16}$, $R^{17}$, $R^{18}$, and $R^{19}$ can be aromatic, i.e., phenyl, n is 1, and p is 1-5, specifically 1-2. In some aspects at least one of $R^{16}$, $R^{17}$, $R^{18}$, $R^{19}$, and X corresponds to a monomer used to form the polycarbonate, e.g., bisphenol A or resorcinol. In another aspect, X is derived especially from resorcinol, hydroquinone, bisphenol A, or diphenylphenol, and $R^{16}$, $R^{17}$, $R^{18}$, $R^{19}$, is aromatic, specifically phenyl. A specific aromatic organophosphorus compound of this type is resorcinol bis(diphenyl phosphate), also known as RDP. Another specific class of aromatic organophosphorus compounds having two or more phosphorus-containing groups are compounds of formula (10)

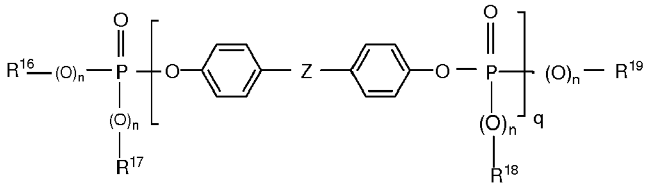

(10)

wherein $R^{16}$, $R^{17}$, $R^{18}$, $R^{19}$, n, and q are as defined for formula (8) and wherein Z is $C_{1-7}$ alkylidene, $C_{1-7}$ alkylene, $C_{5-12}$ cycloalkylidene, -O-, -S-, $-SO_2-$, or -CO-, specifically isopropylidene. A specific aromatic organophosphorus compound of this type is bisphenol A bis(diphenyl phosphate), also known as BPADP, wherein $R^{16}$, $R^{17}$, $R^{18}$, and $R^{19}$ are each phenyl, each n is 1, and q is from 1 to 5, from 1 to 2, or 1.

Organophosphorus compounds containing at least one phosphorus-nitrogen bond includes phosphazenes, phosphorus ester amides, phosphoric acid amides, phosphonic acid amides, phosphinic acid amides, and tris(aziridinyl) phosphine oxide. Phosphazenes (11) and cyclic phosphazenes (12)

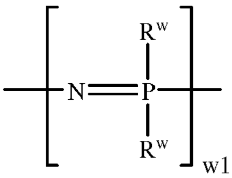 (11) 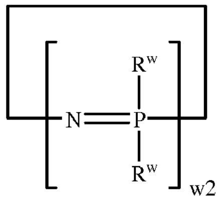 (12)

in particular can used, wherein w1 is 3 to 10,000 and w2 is 3 to 25, specifically 3 to 7, and each $R^w$ is independently a $C_{1-12}$ alkyl, alkenyl, alkoxy, aryl, aryloxy, or polyoxyalkylene group. In the foregoing groups at least one hydrogen atom of these groups can be substituted with a group having an N, S, O, or F atom, or an amino group. For example, each $R^w$ can be a substituted or unsubstituted phenoxy, an amino, or a polyoxyalkylene group. Any given $R^w$ can further be a crosslink to another phosphazene group. Exemplary crosslinks include bisphenol groups, for example bisphenol A groups. Examples include phenoxy cyclotriphosphazene, octaphenoxy cyclotetraphosphazene decaphenoxy cyclopentaphosphazene, and the like. A combination of different phosphazenes can be used. A number of phosphazenes and their synthesis are described in H. R. Allcook, "Phosphorus-Nitrogen Compounds" Academic Press (1972), and J. E. Mark et al., "Inorganic Polymers" Prentice-Hall International, Inc. (1992).

Depending on the particular organophosphorus compound used, the thermoplastic compositions can comprise 0.5 to 15 wt.% or 3.5 to 12 wt.% of the organophosphorus flame retardant, each based on the total weight of the composition. Specifically, the organophosphorus compounds can be bisphenol A bis(diphenyl phosphate), triphenyl phosphate, resorcinol bis(diphenyl phosphate), tricresyl phosphate, or a combination thereof.

The thermoplastic compositions can further comprise a cyclic siloxane and/or a linear siloxane to impart fire/flame-retardant properties. The cyclic siloxane can include those with the general formula below

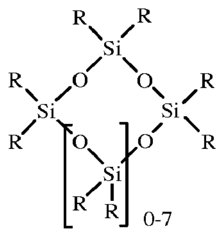

wherein R is each independently $C_{1-36}$ alkyl, fluorinated or perfluorinated $C_{1-36}$ alkyl, $C_{1-36}$ alkoxy, $C_{6-14}$ aryl, aryloxy of 6 to 14 carbon atoms, arylalkoxy of 7 to 36 carbon atoms, or $C_{1-36}$ alkyl-substituted aryl of 6 to 14 carbon atoms. In an embodiment, at least one R may be a phenyl. Examples of cyclic siloxanes include, but not limited to, a cyclic phenyl containing siloxane, octaphenylcyclotetrasiloxane, hexamethylcyclotrisiloxane, octamethylcyclotetrasiloxane, decamethylcyclopentasiloxane, dodecamethylcyclohexasiloxane, trimethyltriphenylcyclotrisiloxane, and tetramethyltetraphenylcyclotetrasiloxane. Octaphenylcyclotetrasiloxane is specifically mentioned.

Linear siloxanes can also be used. The linear siloxanes can be a linear phenyl containing siloxane such as a poly(phenylmethylsiloxane). In an aspect, the thermoplastic compositions contain 0.01% to 1% of a cyclic siloxane, a linear siloxane, or a combination thereof.

Ultraviolet UV absorbing additives include hydroxybenzophenones; hydroxybenzotriazoles; hydroxybenzotriazines; cyanoacrylates; oxanilides; benzoxazinones; 2-(2H-benzotriazol-2-yl)-4-(1,1,3,3-tetramethylbutyl)-phenol (CYASORB™ 5411); 2-hydroxy-4-n-octyloxybenzophenone (CYASORB™ 531); 2-[4,6-bis(2,4-dimethylphenyl)-1,3,5-triazin-2-yl]-5-(octyloxy)-phenol (CYASORB™ 1164); 2,2'-(1,4-phenylene)bis(4H-3,1-benzoxazin-4-one) (CYASORB™ UV-3638); 1,3-bis[(2-cyano-3,3-diphenylacryloyl)oxy]-2,2-bis[[(2-cyano-3, 3-diphenylacryloyl)oxy]methyl]propane (UVINUL™ 3030); 2,2'-(1,4-phenylene)bis(4H-3,1-benzoxazin-4-one); 1,3-bis[(2-cyano-3,3-diphenylacryloyl)oxy] -2,2-bis[[(2-cyano-3,3-diphenylacryloyl)oxy]methyl]propane; phenol, 2-(2H-benzotriazol-2-yl)-4,6-bis(1-methyl-1-phenylethyl)-(TINUVIN™ 234); BCAP bismalonate from Clariant; nano-size inorganic materials such as titanium oxide, cerium oxide, and zinc oxide, all with particle size less than or equal to 100 nanometers;, or combinations comprising at least one of the foregoing UV absorbers. UV absorbers are used in amounts of 0.01 to 5 parts by weight, based on 100 parts by weight of the total composition, excluding any filler.

In an aspect the thermoplastic compositions comprise 5 to 35 weight percent of titanium dioxide, based on the total weight of the thermoplastic compositions.

The thermoplastic compositions can be manufactured by various methods known in the art. For example, powdered polycarbonate, and other optional components are first blended, optionally with any fillers, in a high-speed mixer or by hand mixing. The blend is then fed into the throat of a twin-screw extruder via a hopper. Alternatively, at

least one of the components can be incorporated into the composition by feeding it directly into the extruder at the throat and/or downstream through a sidestuffer, or by being compounded into a masterbatch with a desired polymer and fed into the extruder. The extruder is generally operated at a temperature higher than that necessary to cause the composition to flow. The extrudate can be immediately quenched in a water bath and pelletized. The pellets so prepared can be one-fourth inch long or less as desired. Such pellets can be used for subsequent molding, shaping, or forming.

The disclosed composition may be characterized by specific properties. Accordingly, a drug delivery system comprising the composition of the present disclosure may exhibit certain desirable properties. In one example, the composition may have a low yellowness index and may be heat stable. For example, a molded sample comprising the composition has a yellowness index (YI) of 2.5 or less, 2.0 or less, 1.5 or less, 1.2 or less, or 1.1 or less as measured by ASTM D1925. The composition can further be characterized by a molded sample thereof having an increase in YI ($\Delta$YI) of less than 12, less than 12, or less than 10 after 5,000 hours of heat aging at 130 °C as measured by ASTM D1925. Alternatively, or in addition, the composition can have an increase in YI ($\Delta$YI) of less than 3, less than 2.5, or less than 2 after 2,000 hours of heat aging at 130 °C.

The composition may be transparent in the absence of any light diffusers or other fillers. For example, a molded article of the composition has transmission level greater than or equal to 90.0% at 2.5 millimeter (mm) thickness as measured by ASTM D1003-00, Procedure A, measured, e.g., using a HAZE-GUARD DUAL from BYK-Gardner, using and integrating sphere (0°/diffuse geometry), wherein the spectral sensitivity conforms to the International Commission on Illumination (CIE) standard spectral value under standard lamp D65.

The disclosed thermoplastic compositions can have a glass transition temperature of 155 °C or higher, or from 155 °C to 280 °C, or from 165 °C to 260 °C, or from 185 °C to 230 °C, determined by differential scanning calorimetry (DSC) as per ASTM D3418 with a 20 °C/min heating rate.

The thermoplastic compositions can have excellent transparency. In an aspect, the thermoplastic compositions have a haze less of less than 15%, more preferably less than 10%, more preferably less than 5%, even more preferably less than 1% and a transmission greater than 85%, more preferably greater than 87%, more preferably greater than 89%, even more preferably greater than 90% each measured according to ASTM D1003-00 using the color space CIE1931 (Illuminant C and a 2° observer) at a 2.5 mm thickness. The thermoplastic compositions are molded under standard molding conditions. For example, the thermoplastic compositions are molded at a temperature of 100 °C to 175 °C above the glass transition temperature of the thermoplastic composition for a residence time of 2 to 20 minutes.

The thermoplastic compositions can have excellent transparency in the infrared wavelength range. In an aspect, the compositions have a transmission at wavelength of 1,000 nm of greater than 87.0%, preferably greater than 88.0%, more preferably greater than 89.0%, as measured with Perkin Elmer 950 spectrometer equipped with 15 cm integrated sphere on 2.5 mm. In another aspect, the compositions have a transmission at wavelength of 1,250 nm of greater than 87.0%, preferably greater than an 88.0%, more preferably greater than 89.0%, as measured with Perkin Elmer 950 spectrometer equipped with 15 cm integrated sphere on 2.5 mm. In still another aspect, the compositions have a transmission at wavelength of 1,560 nm of greater than 85.0%, preferably greater than 86.0%, more preferably greater than 87.0%, as measured with Perkin Elmer 950 spectrometer equipped with 15 cm integrated sphere on 2.5 mm.

The thermoplastic compositions can have excellent color. In an aspect, the thermoplastic compositions have a yellowness index of less than 20, more preferably less than 10, more preferably less than 5, more preferably less than 3, as measured by ASTM D1925 on a 2.5 mm plaque. The thermoplastic compositions are molded under standard molding conditions. For example, the thermoplastic compositions are molded at a temperature of 100 °C to 175 °C above the glass transition temperature of the thermoplastic composition for a residence time of 2 to 20 minutes.

The thermoplastic compositions have excellent color stability during exposure for prolonged time at elevated temperatures in the absence of moisture, referred to further as heat ageing. The thermoplastic compositions can have an increase in yellowness index of less than 10, more preferably less than 5, more preferably less than 3, during 2500 hours of heat aging at 140 °C, as measured by ASTM D1925 on a 2.5 mm thick molded plaque. In an aspect, the thermoplastic compositions can have an increase in yellowness index of less than 20, more preferably less than 10, more preferably less than 5, during 2500 hours of heat aging at 150 °C, as measured by ASTM D1925 on a 2.5 mm thick molded plaque. In another aspect, the thermoplastic compositions can have an increase in yellowness index of less than 20, more preferably less than 10, more preferably less than 5, during 1000 hours of heat aging at 160 °C, as measured by ASTM D1925 on a 2.5 mm thick molded plaque. In still another aspect, the thermoplastic compositions can have an increase in yellowness index of less than 20, more preferably less than 10, more preferably less than 5, during 500 hours of heat aging at 170 °C, as measured by ASTM D1925 on a 2.5 mm thick molded plaque.

The thermoplastic compositions have excellent color stability during exposure for prolonged time at elevated temperatures in the presence of moisture, referred to further as hydro ageing. In an aspect, the thermoplastic compositions have an increase in yellowness index of less than 5, more preferably less than 3, more preferably less than 1, after 1000 hours of hydro ageing at 80 °C and 85% relative humidity, as measured by ASTM D1925 on a 2.5 mm thick molded plaque.

The thermoplastic compositions have excellent color stability during exposure for prolonged time to autoclave conditions or multiple cycle of autoclave sterilization. In an aspect, the thermoplastic compositions have an increase in

yellowness index of less than 2, more preferably less than 1, after 100 hours of autoclaving at 121 °C, as measured by ASTM D1925 on a 2.5 mm thick molded plaque. In an aspect, the thermoplastic compositions have an increase in yellowness index of less than 5, more preferably less than 3, more preferably less than 1, after 100 hours of autoclaving at 134 °C, as measured by ASTM D1925 on a 2.5 mm thick molded plaque. In another aspect, the thermoplastic compositions have an increase in yellowness index of less than 10, more preferably less than 5, more preferably less than 3, after 100 hours of autoclaving at 143 °C, as measured by ASTM D1925 on a 2.5 mm thick molded plaque.

Shaped, formed, or molded articles comprising the thermoplastic compositions are also provided. The compositions can be molded into useful shaped articles by a variety of methods, such as injection molding, extrusion, rotational molding, blow molding, and thermoforming. The article can be a molded article, a thermoformed article, an extruded film, an extruded sheet, one or more layers of a multi-layer article, a substrate for a coated article, or a substrate for a metallized article made from the thermoplastic composition.

In some examples, the thermoplastic composition is useful for over-molding for components designated for use within or implantation within the body or at living tissues. As such, the thermoplastic composition may be suitable as a component of an implantation/implanted device or as a component of a device that is configured for use adjacent a surface of living tissues, such as on the skin. The thermoplastic composition is capable of a number of known over-molding applications. As described herein, the thermoplastic compositions having a $T_g$ of at least 150 °C are suitable for autoclaving techniques common in the sterilization of heat and moisture-stable items such as surgical instruments, implanted medical devices.

Advantageously, the articles have no significant part distortion or discoloration when the articles are subjected to a secondary operation such as over-molding, lead-free soldering, low temperature soldering, or coating with high temperature curing, or a combination thereof.

As an example, the disclosed compositions may be useful as for molding plastic objects comprising fewer and/or lower amounts of extractables and leachables. There are a number of safety thresholds as guidelines for minimizing leachables in moldable plastic objects for drug delivery. Safety thresholds allow for a science and risk-based determination of acceptable levels of leachables and can be based on results from preclinical exposure studies, as well as additional safety risk factors that consider, e.g., route of administration, daily exposure, and treatment duration. Such safety thresholds are derived from exposure data and are considered in terms of units of exposure, such as Total Daily Intake (TDI). Thus, any safety threshold is converted into units of concentration (e.g., micrograms per milliliter, μg/ml) so that it can be applied as an analytical threshold in the laboratory. An example of a safety threshold concept that has been practically applied in pharmaceutical development is the Threshold of Toxicological Concern (TTC) approach The TTC concept was adopted by the European Medicines Agency (EMA) to evaluate genotoxic impurities, using an excess cancer risk factor of 105 (1 in 100,000). The EMA's proposed safety threshold for genotoxic impurities using the TTC approach is 1.5 μg/day Total Daily Intake (TDI). Other examples of safety thresholds include the Product Quality Research Institute (PQRI) Safety Concern Threshold (SCT) and Qualification Threshold (QT), derived and proposed for individual organic leachables in Orally Inhaled Nasal Drug Products (OINDP). The SCT is 0.15 μg/day TDI, and the QT is proposed at 5 μg/day TDI for an individual organic leachable. The development of the TTC approach provided a foundation, precedent, and guide for derivation of the PQRI SCT, which incorporates a 10 6 (1 in 1,000,000) risk factor rather than the 10 5 value used for the EMA threshold.

Regarding extractables, the disclosed compositions exhibit minimal levels. For example, the desired polymer, such as a high heat polymer may exhibit certain properties with respect to extractables. In a specific example, granulate of Lexan™ homopolymer (commercially available as HP2REU for medical and pharmaceutical applications) has been examined for extractable compounds. The polymer was subjected to different analytical techniques in order to detect, identify and (semi-)quantify compounds of miscellaneous physicochemical properties. Different extraction methods were applied to release those compounds including: neat headspace analysis (150 °C for 40 min), autoclave extraction with ultra-pure water UPW (121 °C / 60 min), closed vessel extraction with ethanol EtOH (70 °C / 24 h), closed vessel extraction with hexane (60 °C / 24 h); Corresponding blanks (pure solvent/empty headspace vial) were prepared and used as blank reference in the evaluation of the analytical results by Nelson Labs.

The drug delivery systems and related compositions disclosed herein exhibited improvement with respect to levels of extractables. More specifically, in various aspects the drug delivery systems may incorporate compositions exhibiting a reduced a volatile or non-volatile organic compound content. As described herein, volatile or non-volatile organic compound content may refer to the amount of a given volatile, semi-volatile, or non-volatile organic compounds present in a given composition. The disclosed composition may exhibit a volatile or non-volatile organic compound content at least 10 %, at least 20 %, at least 30%, or at least 35% lower as compared to a reference sample of an otherwise identical composition except none of the following characteristics apply: (i) the bisphenol A carbonate units in the copolycarbonate are derived from a bisphenol A monomer having a purity of equal to or greater than 99.70%; or (ii) the bisphenol A homopolycarbonate is present in an amount of greater than zero percent and is derived from a bisphenol A monomer having a purity of equal to or greater than 99.70%; or (iii) the bisphenol A purity of the thermoplastic composition is equal to or greater than 99.60%. Examples presented herein illustrate that a number of screening methods

may be used to determine the volatile or organic components content of a given composition including but not limited to HS-GC/MS analysis.

In various examples, screening methods for the detection of volatile, semi-volatile and non-volatile organic compounds were applied using headspace gas chromatography mass spectrometry HS-GC/MS analysis, GC/MS and high-resolution accurate mass ultra-performance chromatography HRAM-UPLC/MS. Reported results for these screening methods are semi-quantitative. Additionally, inductively coupled plasma optical emission spectrometry ICP/OES and ICP/MS analyses were performed for the analysis of elements and mercury, respectively.

In an example analyzing high heat polycarbonate Elcrin™ HPCI7 ($T_g$ > 155 °C), screening for semi-volatile organic compounds by GC/MS detected no compounds, at concentrations above the reporting limit (5.0 micrograms per gram, μg/g) and different from the blank in the EtOH and hexane extracts. Screening for non-volatile organic compounds by HRAM-UPLC/MS detected 1 compound at a concentration above the reporting limit (5.0 μg/g) and different from the blank in the EtOH extract in atmospheric-pressure chemical ionization APCI- mode. This was the ethyl carbonate derivative of 3,3-bis(4-hydroxyphenyl)-2-phenylisoindolin-1-one monomer at a concentration of 9 μg/g. In APCI+ mode, no differential compounds, were detected. In the hexane extract, no differential compounds were detected above the reporting limit (5.0 μg/g) in both APCI+ and APCI- mode. Analysis for elements by ICP/OES of the UPW extract indicated that no elements were detected above the quantification limit and different from the blank. The foregoing extractables analysis demonstrates that the high heat polymer Elcrin™ HPC17 is particularly useful for overmolding of implantable or surgical devices or in the drug delivery system of the present application.

The present disclosure provides a drug delivery system comprising the disclosed composition. In various embodiments, the drug delivery system comprises a component that comprises the disclosed thermoplastic composition. This component may comprise a housing or another portion of the system. For example, the drug delivery system may comprise a housing, wherein the housing contains a thermoplastic composition comprising: bisphenol-A polycarbonate, wherein a molded article of the composition has transmission level greater than or equal to 90.0% at 2.5 mm thickness as measured by ASTM D1003-00 and a yellow index (YI) less than or equal to 1.5 as measured by ASTM D1925; and optionally a second polycarbonate derived from bisphenol-A, wherein the second polycarbonate is different than the bisphenol-A polycarbonate. A suitable drug delivery system comprising the composition may be selected from inhalers, syringes, injection pens, and auto-injectors or auto-injector devices.

Set forth below are various embodiments of the disclosure.

Embodiment 1. A drug delivery system comprising a housing, wherein the housing contains a thermoplastic composition comprising: a copolycarbonate comprising bisphenol A carbonate units and second carbonate units of the formula

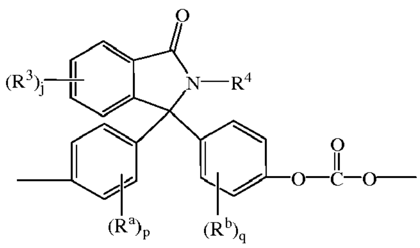

wherein $R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4; and optionally, a bisphenol A homopolycarbonate; wherein one or more of the following conditions apply: the bisphenol A carbonate units in the copolycarbonate are derived from a bisphenol A monomer having a purity of equal to or greater than 99.70%; or the bisphenol A homopolycarbonate is present in an amount of greater than zero percent and is derived from a bisphenol A monomer having a purity of equal to or greater than 99.70%; or the bisphenol A purity of the thermoplastic composition is equal to or greater than 99.60%; and wherein the composition has: a volatile or non-volatile organic compound content that is at least 30% lower than a reference sample of an otherwise identical composition in which none of conditions (i)-(iii) applies, a glass transition temperature of 155 °C or higher, preferably 155 °C to 280 °C, and a molded part of the composition with a thickness of 2.5 mm has a yellowness index (YI) determined according to ASTM D1925 at least 30% lower as compared to a reference sample of an otherwise identical composition except none of conditions (i)-(iii) applies, when both the sample and the reference sample are molded at a temperature of equal to or greater than 330 °C.

Embodiment 2. A drug delivery system comprising a housing, wherein the housing contains a thermoplastic composition comprising: a copolycarbonate comprising bisphenol A carbonate units and second carbonate units of the formula

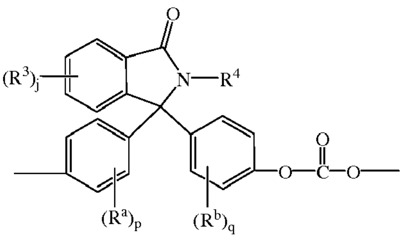

wherein $R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4; and optionally, a bisphenol A homopolycarbonate; wherein one or more of the following conditions apply: the bisphenol A carbonate units in the copolycarbonate are derived from a bisphenol A monomer having a purity of equal to or greater than 99.70%; or the bisphenol A homopolycarbonate is present in an amount of greater than zero percent and is derived from a bisphenol A monomer having a purity of equal to or greater than 99.70%; or the bisphenol A purity of the thermoplastic composition is equal to or greater than 99.60%; wherein the composition has a volatile or non-volatile organic compound content that is at least 30% lower than a reference sample of an otherwise identical composition in which none of conditions (i)-(iii) applies, and wherein the composition has a light transmission of greater than 85% as measured by ASTM D1003-00, or a yellowness index of less than 10 as measured by ASTM D1925, or a combination thereof, wherein the light transition and the yellowness index are measured on a plaque of 2.5 mm molded under at a temperature of 100 °C to 175 °C above the glass transition temperature of the thermoplastic composition for a residence time of 2 to 20 minutes .

Embodiment 3. The drug delivery system of claim 1 or claim 2, wherein in the second carbonate units of the high heat polymer, $R^a$ and $R^b$ are each independently a $C_{1-3}$ alkyl group, $R^3$ is each independently a $C_{1-6}$ alkyl group, $R^4$ is hydrogen, $C_{1-6}$ alkyl or phenyl optionally substituted with 1 to 5 $C_{1-6}$ alkyl groups, and p, q, and j are each independently 0 to 4, preferably, the second carbonate repeating units in the high heat polymer are of the formula

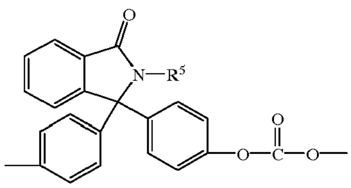

wherein $R^5$ is hydrogen, phenyl or methyl, preferably phenyl.

Embodiment 4. The drug delivery system of any one of claims 1 to 3, wherein the copolycarbonate comprises from 15 mole % to 90 mole % of the bisphenol A carbonate units and 10 mol% to 85 mol% of the second carbonate units, each based on the total number of carbonate units in the copolycarbonate.

Embodiment 5. The drug delivery system of any one of claim 1 to 4, wherein the high heat copolymer further comprises third carbonate units different from the bisphenol A carbonate units and the second carbonate unit, the third carbonate units comprising carbonate units of the formula:

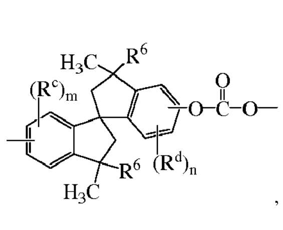 , 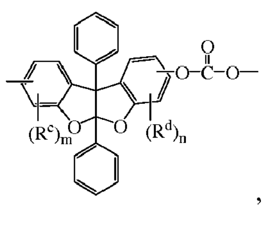 ,

or

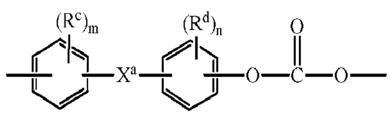

wherein $R^c$ and $R^d$ are each independently a $C_{1-12}$ alkyl, $C_{1-12}$ alkenyl, $C_{3-8}$ cycloalkyl, or $C_{1-12}$ alkoxy, each $R^6$ is

independently $C_{1-3}$ alkyl or phenyl, $X^a$ is a $C_{6-12}$ polycyclic aryl, $C_{3-18}$ mono- or polycycloalkylene, $C_{3-18}$ mono- or polycycloalkylidene, $-(Q^1)_x-G-(Q^2)_y-$ group wherein $Q^1$ and $Q^2$ are each independently a $C_{1-3}$ alkylene, G is a $C_{3-10}$ cycloalkylene, x is 0 or 1, and y is 1, or $-C(P^1)(P^2)-$ wherein $P^1$ is $C_{1-12}$ alkyl and $P^2$ is $C_{6-12}$ aryl, and m and n are each independently 0 to 4, or a combination thereof. The thermoplastic composition of any one of claims 1 to 8, wherein the copolycarbonate comprises less than 2 ppm of each of triethyl amine, calcium ions, magnesium ions, potassium ions, iron ions, and chloride ions.

Embodiment 6. The drug delivery system of any one of claims 1 to 5, comprising 10 to 90 wt.% of the bisphenol A homopolycarbonate based on the total weight of the composition.

Embodiment 7. The drug delivery system of any one of claims 1 to 6, wherein the bisphenol A purity of the thermoplastic composition is equal to or greater than 99.60%, measured using high performance liquid chromatography.

Embodiment 8. The drug delivery system of any one of claims 1 to 7, further comprising 1 ppm to 40 ppm of an acid additive.

Embodiment 9. The drug delivery system of any one of claims 1-8, wherein the thermoplastic composition is suitable for overmolding components for medical devices or implants.

Embodiment 10. The drug delivery system of any one of claims 1-9, wherein the thermoplastic composition exhibits an autoclave resistance to at least 100 hours of autoclaving at 120 °C.

Embodiment 11. A drug delivery system of claims 1 to 10, wherein the drug delivery system is selected from at least one of the following medical devices: inhaler, syringe, injection pen, and auto-injector.

Embodiment 12. A drug delivery system comprising a housing, wherein the housing contains a thermoplastic composition comprising: bisphenol-A polycarbonate, wherein the composition has a volatile or non-volatile organic compound content that is at least 30% lower than a reference sample of an otherwise identical composition in which none of conditions (i)-(iii) applies and wherein a molded article of the composition has transmission level greater than or equal to 90.0% at 2.5 mm thickness as measured by ASTM D1003-00 and a yellow index (YI) less than or equal to 1.5 as measured by ASTM D1925; and optionally a second polycarbonate derived from bisphenol-A, wherein the second polycarbonate is different than the bisphenol-A polycarbonate.

Embodiment 13. The drug delivery system as in Claim 12, wherein the bisphenol-A polycarbonate comprises less than or equal to 150 ppm free hydroxyl groups.

Embodiment 14. The drug delivery system as in Claim 12 to Claim 13, wherein the bisphenol-A polycarbonate comprises sulfur in an amount less than or equal to 2 ppm sulfur.

Embodiment 15. The drug delivery system as in Claim 12 to Claim 14, wherein the molded article comprises an increase in yellow index (YI) of less than 2 during 2,000 hours of heat aging at 130 °C.

Embodiment 16. The drug delivery system as in Claim 12 to 15, wherein the housing is suitable for use in autoclaving techniques.

Embodiment 17. A drug delivery system of Claim 12 to Claim 16, wherein the drug delivery system is selected from at least one of the following medical devices: inhaler, syringe, injection pen, and auto-injector.

Embodiment 18. An article, wherein the article a molded article, a thermoformed article, an extruded film, an extruded sheet, one or more layers of a multi-layer article, a substrate for a coated article, or a substrate for a metallized article is made from the copolymer or composition of any one or more of claims 1 to 17.

Embodiment 19. The article of claim 18, wherein the article has no significant part distortion or discoloration when the article is subjected to a secondary operation comprising over-molding, lead-free soldering, low temperature soldering, or coating, or a combination thereof.

Embodiment 20. The article of claim 18 or 19, wherein the article is a lens or cover for lighting devices, a lens holder, motor vehicle headlights, automotive rear lights, automotive fog lights, flash lights, cameras, mobile phone cameras; a light guide, a substrate film, a signal indicator, a waveguide element, a reflector, a collimator, a housing for a light source, a lamp bezel, a lamp holder, a lamp cover, a display screen, glazing, a safety goggle, a visor, a medical device, a face shield, an optical fiber, a fuse, a part of a domestic appliance, a window or door for domestic appliances, computer and business machine housings such as housings for monitors, handheld electronic device housings such as housings for cell phones, electrical connectors, a fire shield, a food tray, a packaging film, an animal cage, a tray, an optical film, a light bulb, or a film laminate.

The singular forms "a," "an," and "the" include plural referents unless the context clearly dictates otherwise. "Or" means "and/or." The endpoints of all ranges directed to the same component or property are inclusive and independently combinable. Unless defined otherwise, technical and scientific terms used herein have the same meaning as is commonly understood by one of skill in the art to which this invention belongs. As used herein, a "combination" is inclusive of blends, mixtures, alloys, reaction products, and the like. A "combination thereof' includes any combination comprising at least one of the listed components or properties optionally together with a like component or property not listed.

Compounds are described using standard nomenclature. For example, any position not substituted by any

indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group.

As used herein, the term "hydrocarbyl" and "hydrocarbon" refers broadly to a substituent comprising carbon and hydrogen, optionally with 1 to 3 heteroatoms, for example, oxygen, nitrogen, halogen, silicon, sulfur, or a combination thereof; "alkyl" refers to a straight or branched chain, saturated monovalent hydrocarbon group; "alkylene" refers to a straight or branched chain, saturated, divalent hydrocarbon group; "alkylidene" refers to a straight or branched chain, saturated divalent hydrocarbon group, with both valences on a single common carbon atom; "alkenyl" refers to a straight or branched chain monovalent hydrocarbon group having at least two carbons joined by a carbon-carbon double bond; "cycloalkyl" refers to a non-aromatic monovalent monocyclic or multicylic hydrocarbon group having at least three carbon atoms; "cycloalkylene" refers to a divalent radical formed by the removal of two hydrogen atoms from two different carbon atoms on one or more rings of a cycloalkyl group; "aryl" refers to an aromatic monovalent group containing only carbon in the aromatic ring or rings; "arylene" refers to an aromatic divalent group containing only carbon in the aromatic ring or rings; "alkylaryl" refers to an aryl group that has been substituted with an alkyl group as defined above, with 4-methylphenyl being an exemplary alkylaryl group; "arylalkyl" refers to an alkyl group that has been substituted with an aryl group as defined above, with benzyl being an exemplary arylalkyl group; "acyl" refers to an alkyl group as defined above with the indicated number of carbon atoms attached through a carbonyl carbon bridge (-C(=O)-); "alkoxy" refers to an alkyl group as defined above with the indicated number of carbon atoms attached through an oxygen bridge (-O-); and "aryloxy" refers to an aryl group as defined above with the indicated number of carbon atoms attached through an oxygen bridge (-O-).

As used herein, "drug delivery system" may describe a mechanism or means by which a drug or other therapeutic is delivered to a subject patient. A drug delivery system may include a housing in which an amount of a subject drug or therapeutic is stored or contained for delivery to a subject. An example of a delivery system may comprise transdermal delivery which is accomplished by exposing a source of a drug or therapeutic to a subject's skin for an extended period of time. Typically, the drug is incorporated in a housing or reservoir from which it is released onto the patient's skin. The rate of release can be controlled by a membrane, for example, placed between the reservoir and the skin, by diffusion from a monolithic device, or by the skin itself serving as a rate-controlling barrier in the delivery system. Drug delivery systems may include inhalers, syringes, injection pens, and auto-injectors or auto-injector devices.

Unless otherwise indicated, each of the foregoing groups can be unsubstituted or substituted, provided that the substitution does not significantly adversely affect synthesis, stability, or use of the compound. The term "substituted" as used herein means that at least one hydrogen on the designated atom or group is replaced with another group, provided that the designated atom's normal valence is not exceeded. When the substituent is oxo (i.e., =O), then two hydrogens on the atom are replaced. Combinations of substituents and/or variables are permissible provided that the substitutions do not significantly adversely affect synthesis or use of the compound. Groups that can be present on a substituted position include (-$NO_2$), cyano (-CN), hydroxy (-OH), halogen, thiol (-SH), thiocyano (-SCN), $C_{2-6}$ alkanoyl (e.g., acyl ($H_3CC(=O)$-); carboxamido; $C_{1-6}$ or $C_{1-3}$ alkyl, cycloalkyl, alkenyl, and alkynyl (including groups having at least one unsaturated linkages and from 2 to 8, or 2 to 6 carbon atoms); $C_{1-6}$ or $C_{1-3}$ alkoxy; $C_{6-10}$ aryloxy such as phenoxy; $C_{1-6}$ alkylthio; $C_{1-6}$ or $C_{1-3}$ alkylsulfinyl; C1-6 or $C_{1-3}$ alkylsulfonyl; aminodi($C_{1-6}$ or $C_{1-3}$)alkyl; $C_{6-12}$ aryl having at least one aromatic rings (e.g., phenyl, biphenyl, naphthyl, or the like, each ring either substituted or unsubstituted aromatic); $C_{7-19}$ arylalkyl having 1 to 3 separate or fused rings and from 6 to 18 ring carbon atoms; or arylalkoxy having 1 1 to 3 separate or fused rings and from 6 to 18 ring carbon atoms.

While typical embodiments have been set forth for the purpose of illustration, the foregoing descriptions should not be deemed to be a limitation on the scope herein. Accordingly, various modifications, adaptations, and alternatives can occur to one skilled in the art without departing from the spirit and scope herein.

It will be apparent to those skilled in the art that various modifications and variations can be made in the present disclosure without departing from the scope or spirit of the disclosure. Other embodiments of the disclosure will be apparent to those skilled in the art from consideration of the specification and practice of the disclosure disclosed herein. It is intended that the specification and examples be considered as exemplary only, with a true scope and spirit of the disclosure being indicated by the following claims.

The patentable scope of the disclosure is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

**1.** A drug delivery system comprising a component, wherein the component contains a thermoplastic composition

comprising:

a copolycarbonate comprising bisphenol A carbonate units and second carbonate units of the formula

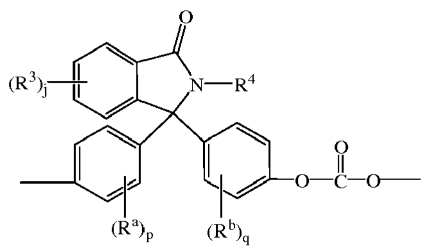

wherein

$R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4; and

optionally, a bisphenol A homopolycarbonate;
wherein one or more of the following conditions apply:

(i) the bisphenol A carbonate units in the copolycarbonate are derived from a bisphenol A monomer having a purity of equal to or greater than 99.70%; or
(ii) the bisphenol A homopolycarbonate is present in an amount of greater than zero percent and is derived from a bisphenol A monomer having a purity of equal to or greater than 99.70%; or
(iii) the bisphenol A purity of the thermoplastic composition is equal to or greater than 99.60%; and

wherein the composition has:

a glass transition temperature of 155 °C or higher, preferably 155 °C to 280 °C,
a volatile or non-volatile organic compound content that is at least 30% lower than a reference sample of an otherwise identical composition in which none of conditions (i)-(iii) applies;
a molded part of the composition with a thickness of 2.5 mm has a yellowness index (YI) determined according to ASTM D1925 at least 30% lower as compared to a reference sample of an otherwise identical composition except none of conditions (i)-(iii) applies, when both the sample and the reference sample are molded at a temperature of equal to or greater than 330 °C.

**2.** A thermoplastic composition comprising: a copolycarbonate comprising bisphenol A carbonate units and second carbonate units of the formula

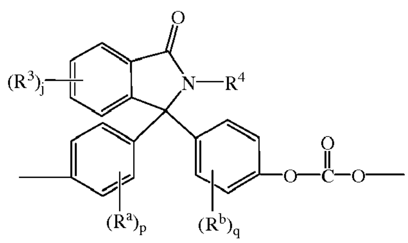

wherein

$R^a$ and $R^b$ are each independently a $C_{1-12}$ alkyl, $C_{1-12}$ alkenyl, $C_{3-8}$ cycloalkyl, or $C_{1-12}$ alkoxy, each $R^3$ is independently a $C_{1-6}$ alkyl, $R^4$ is hydrogen, $C_{2-6}$ alkyl or phenyl optionally substituted with 1 to 5 $C_{1-6}$ alkyl groups, p, q, and j are each independently 0 to 4; and

optionally, a bisphenol A homopolycarbonate;
wherein one or more of the following conditions apply:

(i) the bisphenol A carbonate units in the copolycarbonate are derived from a bisphenol A monomer having a purity of equal to or greater than 99.70%; or
(ii) the bisphenol A homopolycarbonate is present in an amount of greater than zero percent and is derived from a bisphenol A monomer having a purity of equal to or greater than 99.70%; or
(iii) the bisphenol A purity of the thermoplastic composition is equal to or greater than 99.60%; and

wherein the composition has a light transmission of greater than 85% as measured by ASTM D1003-00, or a yellowness index of less than 10 as measured by ASTM D1925, or a combination thereof, wherein the light transition and the yellowness index are measured on a plaque of 2.5 mm molded under at a temperature of 100 °C to 175 °C above the glass transition temperature of the thermoplastic composition for a residence time of 2 to 20 minutes and wherein the composition has a volatile or non-volatile organic compound content that is at least 30% lower than a reference sample of an otherwise identical composition in which none of conditions (i)-(iii) applies.

**3.** The thermoplastic composition of claim 1 or claim 2, wherein in the second carbonate units of the high heat polymer,

$R^a$ and $R^b$ are each independently a $C_{1-3}$ alkyl group,
$R^3$ is each independently a $C_{1-6}$ alkyl group,
$R^4$ is hydrogen, $C_{1-6}$ alkyl or phenyl optionally substituted with 1 to 5 $C_{1-6}$ alkyl groups, and
p, q, and j are each independently 0 to 4,

preferably, the second carbonate repeating units in the high heat polymer are of the formula

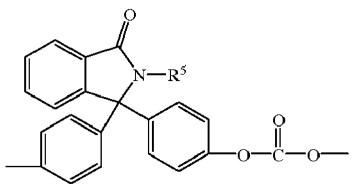

wherein $R^5$ is hydrogen, phenyl or methyl, preferably phenyl.

**4.** The thermoplastic composition of any one of claims 1 to 3, wherein the copolycarbonate comprises from 15 mole % to 90 mole % of the bisphenol A carbonate units and 10 mol% to 85 mol% of the second carbonate units, each based on the total number of carbonate units in the copolycarbonate.

**5.** The thermoplastic composition of any one of claim 1 to 4, wherein the high heat copolymer further comprises third carbonate units different from the bisphenol A carbonate units and the second carbonate unit, the third carbonate units comprising carbonate units of the formula:

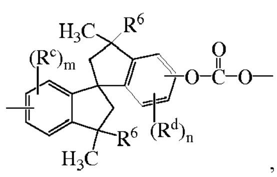 , 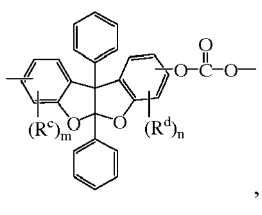 , or 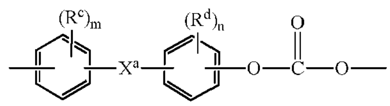

wherein

$R^c$ and $R^d$ are each independently a $C_{1-12}$ alkyl, $C_{1-12}$ alkenyl, $C_{3-8}$ cycloalkyl, or $C_{1-12}$ alkoxy,
each $R^6$ is independently $C_{1-3}$ alkyl or phenyl,
$X^a$ is a $C_{6-12}$ polycyclic aryl, $C_{3-18}$ mono- or polycycloalkylene, $C_{3-18}$ mono- or polycycloalkylidene, $-(Q^1)_x-G-(Q^2)_y-$ group wherein $Q^1$ and $Q^2$ are each independently a $C_{1-3}$ alkylene, G is a $C_{3-10}$ cycloalkylene, x is 0 or 1, and y is 1,

or
$-C(P^1)(P^2)-$ wherein $P^1$ is $C_{1-12}$ alkyl and $P^2$ is $C_{6-12}$ aryl, and

m and n are each independently 0 to 4, or a combination thereof. The thermoplastic composition of any one of claims 1 to 8, wherein the copolycarbonate comprises less than 2 ppm of each of triethyl amine, calcium ions, magnesium ions, potassium ions, iron ions, and chloride ions.

**6.** The thermoplastic composition of any one of claims 1 to 5, comprising 10 to 90 wt.% of the bisphenol A homopolycarbonate based on the total weight of the composition.

**7.** The thermoplastic composition of any one of claims 1 to 6, wherein the bisphenol A purity of the thermoplastic composition is equal to or greater than 99.60%, measured using high performance liquid chromatography.

**8.** The thermoplastic composition of any one of claims 1 to 7, further comprising 1 ppm to 40 ppm of an acid additive.

**9.** The thermoplastic composition of any one of claims 1-8, wherein the thermoplastic composition exhibits an autoclave resistance to at least 100 hours of autoclaving at 120 °C.

**10.** A thermoplastic composition of claims 1 to 9, wherein the thermoplastic composition is suitable for overmolding components of medical devices or implants and wherein the medical devices or implants are selected from one or more of an: inhaler, syringe, injection pen, and auto-injector.

**11.** A drug delivery system comprising a housing, wherein the housing contains a thermoplastic composition comprising: bisphenol-A polycarbonate, wherein the composition has a volatile or non-volatile organic compound content that is at least 30% lower than a reference sample of an otherwise identical composition in which none of conditions (i)-(iii) applies and wherein a molded article of the composition has transmission level greater than or equal to 90.0% at 2.5 mm thickness as measured by ASTM D1003-00 and a yellow index (YI) less than or equal to 1.5 as measured by ASTM D1925; and optionally a second polycarbonate derived from bisphenol-A, wherein the second polycarbonate is different than the bisphenol-A polycarbonate.

**12.** The drug delivery system as in Claim 11, wherein the bisphenol-A polycarbonate comprises less than or equal to 150 ppm free hydroxyl groups.

**13.** The drug delivery system as in Claim 11 to Claim 12, wherein the bisphenol-A polycarbonate comprises sulfur in an amount less than or equal to 2 ppm sulfur.

**14.** The drug delivery system as in Claim 11 to Claim 13, wherein the molded article comprises an increase in yellow index (YI) of less than 2 during 2,000 hours of heat aging at 130 °C.

**15.** An article, wherein the article a molded article, a thermoformed article, an extruded film, an extruded sheet, one or more layers of a multi-layer article, a substrate for a coated article, or a substrate for a metallized article is made from the copolymer or composition of any one or more of claims 1 to 14.

European Patent Office / Europäisches Patentamt / Office européen des brevets

# EUROPEAN SEARCH REPORT

Application Number
EP 19 19 3369

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2017/203496 A1 (SABIC GLOBAL TECHNOLOGIES BV [NL]) 30 November 2017 (2017-11-30) * examples 1-22 * * tables 1-10 * | 1-15 | INV. A61L31/06 C08G64/12 C08L69/00 |
| X | US 2019/169368 A1 (VAN DER MEE MARK ADRIANUS JOHANNES [NL] ET AL) 6 June 2019 (2019-06-06) * tables 1-12 * * claims 1-27 * | 1-15 | |
| X | WO 2018/020425 A1 (SABIC GLOBAL TECHNOLOGIES BV [NL]) 1 February 2018 (2018-02-01) * claims 1-21 * * tables 1-12 * * paragraph [[0092]] - paragraph [[0095]] * | 1-15 | |

TECHNICAL FIELDS SEARCHED (IPC)

A61L
C08G
C08L

The present search report has been drawn up for all claims

1

| Place of search | Date of completion of the search | Examiner |
|---|---|---|
| The Hague | 2 December 2019 | Popescu, Teodora |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 19 3369

This annex lists the patent family members relating to the patent documents cited in the above-mentioned European search report. The members are as contained in the European Patent Office EDP file on
The European Patent Office is in no way liable for these particulars which are merely given for the purpose of information.

02-12-2019

| Patent document cited in search report | Publication date | Patent family member(s) | Publication date |
|---|---|---|---|
| WO 2017203496 A1 | 30-11-2017 | CN 109153847 A | 04-01-2019 |
| | | CN 109153848 A | 04-01-2019 |
| | | EP 3464469 A1 | 10-04-2019 |
| | | EP 3464470 A1 | 10-04-2019 |
| | | US 2019203043 A1 | 04-07-2019 |
| | | US 2019300701 A1 | 03-10-2019 |
| | | WO 2017203496 A1 | 30-11-2017 |
| | | WO 2017203497 A1 | 30-11-2017 |
| US 2019169368 A1 | 06-06-2019 | CN 109153819 A | 04-01-2019 |
| | | CN 109196048 A | 11-01-2019 |
| | | EP 3380553 A1 | 03-10-2018 |
| | | EP 3380563 A1 | 03-10-2018 |
| | | JP 2019517602 A | 24-06-2019 |
| | | JP 2019517682 A | 24-06-2019 |
| | | US 2019169368 A1 | 06-06-2019 |
| | | US 2019218391 A1 | 18-07-2019 |
| | | WO 2017203493 A1 | 30-11-2017 |
| | | WO 2017203495 A1 | 30-11-2017 |
| WO 2018020425 A1 | 01-02-2018 | CN 109415534 A | 01-03-2019 |
| | | EP 3487921 A1 | 29-05-2019 |
| | | US 2019276665 A1 | 12-09-2019 |
| | | WO 2018020425 A1 | 01-02-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017203496 A
- WO 2013175448 A1
- WO 2014072923 A1


### Non-patent literature cited in the description


- **NOWAKOWSKA et al.** *Polish J. Appl. Chem.,* 1996, vol. XI (3), 247-254
- **H. R. ALLCOOK.** Phosphorus-Nitrogen Compounds. Academic Press, 1972
- **J. E. MARK et al.** Inorganic Polymers. Prentice-Hall International, Inc, 1992